# EUROPEAN PATENT SPECIFICATION

(11) **EP 3 636 769 B1**
(45) Date of publication and mention of the grant of the patent: **18.01.2023**
(21) Application number: 18792022.8
(22) Date of filing: 24.05.2018
(51) Int. Cl.: C12Q 1/68, C12N 15/10

(54) **SAMPLE NUCLEIC ACID MEASUREMENT TEST KIT, REAGENT, AND APPLICATION THEREOF**
TESTKIT ZUR PROBENNUKLEINSÄUREMESSUNG, REAGENZ UND ANWENDUNG DAVON
KIT DE TEST DE MESURE D'ACIDE NUCLÉIQUE DANS UN ÉCHANTILLON, RÉACTIF ET APPLICATION ASSOCIÉ

(43) Date of publication of application: 15.04.2020
(73) Proprietor: Leadway (HK) Limited, Sheung Wan, Hong Kong 999077 (CN)
(72) Inventor: ZHANG, Taisong, Zhejiang 310030 (CN); CHEN, Feifei, Zhejiang 310030 (CN); ZHANG, Pan, Zhejiang 310030 (CN); WANG, Shanchen, Zhejiang 310030 (CN)
(74) Representative: Schiweck Weinzierl Koch Patentanwälte Partnerschaft mbB
(86) International application number: PCT/CN2018/088121
(87) International publication number: WO 2018/196879

(56) References cited:
- CN-A- 102 242 192
- CN-A- 102 321 618
- CN-A- 102 559 933
- CN-A- 104 293 913
- WALSH P S ET AL: "CHELEX 100 AS A MEDIUM FOR SIMPLE EXTRACTION OF DNA FOR PCR-BASED TYPING FROM FORENSIC MATERIAL", BIOTECHNIQUES, NATICK, MA, US, vol. 10, no. 4, 1 April 1991 (1991-04-01), pages 506-513, XP001022477,
- Changhyun Roh ET AL: "Extracting eDNA From Soil and Sludge Samples 97 Comparative Study of Methods for Extraction and Purification of Environmental DNA From Soil and Sludge Samples", Applied Biochemistry and Biotechnology, 1 January 2006 (2006-01-01), XP055156056, Retrieved from the Internet: URL:http://rd.springer.com/content/pdf/10. 1385/ABAB:134:2:97.pdf [retrieved on 2014-12-01]
- SUENAGA E ET AL: "Evaluation of three methods for effective extraction of DNA from human hair", JOURNAL OF CHROMATOGRAPHY B: BIOMEDICAL SCIENCES & APPLICATIONS, ELSEVIER, AMSTERDAM, NL, vol. 820, no. 1, 5 June 2005 (2005-06-05), pages 137-141, XP027627313, ISSN: 1570-0232 [retrieved on 2005-06-05]

## Description

### Field of the Invention

The present invention belongs to the field of bioscience and biotechnology, and particularly relates to a method for detecting nucleic acid in a sample, and a sample nucleic acid detection kit suitable for said method.

### Background of the Invention

With the rapid development of molecular biology technology, it has expanded into various fields such as biology, medicine, botany, genetics and zoology. As a major breakthrough in molecular biology technology, polymerase chain reaction (PCR) technology has high sensitivity, high specificity and rapid time-saving feature, and has been widely used in human and animal disease diagnosis (such as prenatal diagnosis, newborn screening and genetic metabolic disease detection), forensic detection, kinship analysis, seed purity identification, molecular marker-assisted breeding, gene mapping and genetically modified organism detection, etc.

The detection using PCR technology mainly involves three aspects: nucleic acid extraction, nucleic acid amplification and amplification product detection. The quality and quantity of extracted nucleic acid generally directly affect the success of the PCR reaction. Therefore, a large number of PCR-based detection methods (such as fluorescent PCR, DNA sequencing, and RNA sequencing, etc.) require extraction and purification of DNA and/or RNA nucleic acid molecules in biological samples (such as serum, plasma, whole blood, urine, vaginal secretion, saliva, feces, cotton swab, epithelial exfoliated cells, fresh tissue and paraffin tissue section, etc.) before amplification.

The extraction and purification of nucleic acids in biological samples have been a time-consuming and tedious process for a long time, which has seriously slowed down the detection speed. Especially in the terms of extremely small amount of biological samples, the low extraction recovery of trace DNA in the sample by conventional nucleic acid extraction methods is the main cause of the poor subsequent PCR amplification effect. Therefore, establishing a rapid DNA extraction method is crucial to improve the efficiency of PCR detection.

There are many extraction methods according to different natures of the nucleic acids. One of the relatively common methods is the Chelex-100-based boiling method, i.e. Chelex-100 boiling method. In this boiling method, Chelex-100 is a chemical ion chelating resin composed of styrene and divinyl benzene copolymer, wherein its suspension solution in the conditions of basic environment (pH 10 to 14) and 100 °C, can cause cell membrane rupture and release DNA from the nucleus after denaturation, and Chelex-100 can bind polyvalent cations with high selectivity, remove polyvalent metal ions in samples and buffers, and avoid degradation of nucleic acid template in the boiling process. In addition, it can effectively remove non-nucleic acid organics, has the advantages of economy, simplicity, high efficiency, etc., and can reduce the loss of nucleic acid in the extraction process, and has been widely used in the nucleic acid extraction of the forensic evidence materials such as trace blood, tissue plaque, seminal stain, hair and bones.

The Chelex-100 boiling method mainly comprises three steps: concentrating biological samples; adding a lysis solution, and boiling at high temperature; centrifuging at high speed to obtain the supernatant, which is the extracted nucleic acid solution. In the boiling method, the lysis solution contains NaOH or KOH in addition to Chelex-100, to ensure that its pH is maintained at 10-14. This also results in the extracted nucleic acid solution maintaining strong alkalinity. If this strong alkalinity is not neutralized and the nucleic acid solution is directly mixed with the PCR reaction solution for amplification reaction, inefficient PCR amplification reaction will occur. In order to solve this problem, in the Chinese patent application CN201110295395.9, 200 mM Tris-HCl buffer (pH 6.8 to 7.5) was added for neutralization after the lysis solution addition and high-temperature boiling, thereby lowering the pH in the extracted nucleic acid solution. However, when the nucleic acid in the biological sample is extracted by this method, the neutralization solution needs to be manually added for neutralization, and the operation step is somewhat cumbersome, and if the operator forgets to add the neutralization solution, the PCR amplification efficiency will be lowered, which will affect the detection result.

Walsh et al. (Biotechniques, vol. 10, no. 4, 1991) also uses Chelex-100 as a medium for simple extraction of DNA for PCR-based typing from forensic material.

In addition, another commonly used nucleic acid extraction method is the alkaline lysis method. The Chinese patent application CN201210242862.6 utilizes a strong alkaline solution (such as NaOH or KOH solution) to lyse the cells in a biological sample at a high temperature, release nucleic acid, and then add an acidic buffer for neutralization, and centrifuge to obtain the extracted nucleic acid solution. Similarly, when the nucleic acid in the biological sample is extracted by this method, the neutralization solution needs to be manually added for neutralization, and the operation step is somewhat cumbersome and if the operator forgets to add the neutralization solution, the PCR amplification efficiency will be lowered, which will affect the detection result.

Regardless of the Chelex-100 boiling method or the alkaline lysis method is used, when the nucleic acid in the sample is extracted and detected in the prior art, there is a problem: the detection of HBV DNA which may exist in the clinical blood or plasma sample is taken as an example, and the strong alkaline nucleic acid extraction solution is used to extract the nucleic acid in the clinical blood or plasma sample, to obtain the extracted nucleic acid solution, and then 2 to 5 µl of the extracted nucleic acid solution is added to about 35 µl of the PCR reaction solution for mixing, because the volume of the added extracted nucleic acid solution is small, so it has little effect on the mixed PCR reaction solution. However, when the volume of the added extracted nucleic acid solution is large, for example, 20 µl of the extracted nucleic acid solution is added to 20 µl of PCR reaction solution for mixing, although the total volume is still 40 µl, the volume ratio of the extracted nucleic acid solution is significantly increased, which affects the mixed PCR reaction solution, resulting in a high pH value, thereby affecting subsequent PCR amplification reaction efficiency, and reducing the detection sensitivity and accuracy of the sample nucleic acid.

In addition, after the nucleic acid in the sample is extracted by the conventional weak basic nucleic acid lysis solution, when the volume of the extracted nucleic acid solution added to the PCR reaction solution is low (usually 2 to 5 µl), although nucleic acid in the sample can be detected, the detection sensitivity is low; however, when the volume of the added extracted nucleic acid solution is large, for example, 20 µl of the extracted nucleic acid solution is added to 20 µl of the PCR reaction solution for mixing, wherein the efficiency of the weak basic nucleic acid lysis solution to extract the sample nucleic acid is poor, and the large volume of nucleic acid solution added is likely to contain more impurities and inhibitors for PCR amplification reaction, which may result in the PCR amplification reaction cannot proceed normally, or although the PCR amplification reaction may proceed, the detection sensitivity and accuracy of the sample nucleic acid will be reduced.

### Summary of the Invention

The invention is defined by the appended claims.

Regarding of the deficiencies of the prior art, the present disclosure provides a reagent, and the present invention provides a method and a kit for detecting a sample nucleic acid, the reagent, the method and the kit all use a strong alkaline nucleic acid extraction solution to extract nucleic acid in a sample, and then the extracted sample nucleic acid is added to the PCR reaction solution for neutralization, and nucleic acid amplification and detection are performed simultaneously, so that a step of neutralizing the extracted sample nucleic acid is unnecessary when the sample nucleic acid is extracted, thereby the detection time will be significantly saved and is also convenient for semi-automatic or fully automated processing, improving the efficiency of PCR amplification reaction, increasing the sensitivity of sample nucleic acid detection and improving the accuracy of nucleic acid detection.

Because the components in serum, plasma, whole blood, sputum, urine, genital tract secretion, tear, feces, fresh tissue, spinal fluid, and paraffin tissue section are very complex, if the nucleic acid in the sample is directly detected, the detection will be interfered by other components, so some samples need to be pretreated and preserved, and then the extracted sample nucleic acids are detected.

For different samples, their pretreatment methods are different, for example, whole blood contains red blood cells, so when nucleic acid is extracted, it can be treated with a common red blood cell lysis solution, and then sample nucleic acid is extracted by using the nucleic acid extraction solution used in the present invention; the sputum contains a large amount of mucin and impurities, so when nucleic acid is extracted, the sample is pretreated: after NaOH liquefaction, mucin is removed usually with sodium citrate, 0.5% N-acetyl-L-cysteine (NALC) or dithiothreitol (DTT), and then the sample nucleic acid is extracted by using the nucleic acid extraction solution used in the present invention; the fresh tissue is pretreated by the steps: first washing the tissue with physiological saline, then performing mashing or chopping, adding proteinase K for digestion, then using the nucleic acid extraction solution used in the present invention to extract the sample nucleic acid; the paraffin tissue section is pretreated by the steps: first de-waxing the tissue section with a de-waxing agent such as xylene, then adding proteinase K for digestion, then using the nucleic acid extraction solution used in the present invention to extract the sample nucleic acid.

The present disclosure provides a sample nucleic acid detection reagent, comprising a nucleic acid extraction solution and a PCR reaction solution, the nucleic acid extraction solution comprising an alkali metal hydroxide 10 mM to 100 mM, Tris-HCl 1 mM to 25 mM, Chelex-100 2% to 5% (w/v), EDTA 0.1% to 0.4% (w/v), the pH of the nucleic acid extraction solution is 10.5 to 12.5; and the PCR reaction solution comprises Tris-HCl 5 mM to 75 mM, and the pH of the PCR reaction solution is 8.0 to 9.0, wherein the PCR reaction solution is able to neutralize the pH of the nucleic acid extraction solution.

It is described herein that the alkali metal hydroxide is NaOH or KOH.

It is described herein that the sample nucleic acid detection reagent further comprises a concentration solution.

It is described herein that the concentration solution comprises 2.5% to 4.5% (w/w) of NaCl and 10% to 13% (w/v) of PEG6000.

It is described herein that the pH of the PCR reaction solution is 8.1 to 8.7.

It is described herein that the pH of the PCR reaction solution is 8.1 to 8.5.

It is described herein that the pH of the PCR reaction solution is 8.1.

The present disclosure also provides use of the sample nucleic acid detection reagent for detecting sample nucleic acid, such as HBV nucleic acid.

It is described herein that the sample nucleic acid is from HBV, human B27, HPV 6/11, TB or HCMV.

It is described herein that the sample nucleic acid comprises HBV nucleic acid; in the nucleic acid extraction solution, the concentration of the alkali metal hydroxide is 25 mM, the concentration of Tris-HCl is 1 mM, and the Chelex-100 concentration is 2% (w/v) and the EDTA concentration is 0.1% (w/v), the pH of the nucleic acid extraction solution is 11.5; in the PCR reaction solution, the concentration of Tris-HCl is 10 mM, and the pH of the PCR reaction solution is 8.1.

The present invention provides a method for detecting nucleic acid in a sample, comprising: (1) extracting nucleic acid in a sample by using a nucleic acid extraction solution to obtain a sample nucleic acid solution; (2) adding the sample nucleic acid solution obtained in (1) to a PCR reaction solution for neutralization to obtain a mixed solution, and using the mixed solution to perform amplification reaction and detect nucleic acid in the sample, wherein the nucleic acid extraction solution comprises an alkali metal hydroxide 10 mM to 100 mM, Tris-HCl 1 mM to 25 mM, Chelex-100 2% to 5% (w/v), EDTA 0.1% to 0.4% (w/v), the pH of the nucleic acid extraction solution is 10.5 to 12.5; and the PCR reaction solution comprises Tris-HCl 5 mM to 75 mM, the pH of the PCR reaction solution is 8.0 to 9.0; and the pH of the mixed solution is 8.0 to 9.0.

Preferably, the alkali metal hydroxide is NaOH or KOH.

Preferably, prior to step (1), the sample is concentrated with a concentration solution.

Preferably, the concentration solution comprises 2.5% to 4.5% (w/w) of NaCl and 10% to 13% (w/v) of PEG6000.

Preferably, the pH of the PCR reaction solution is 8.1 to 8.7.

Preferably, the pH of the PCR reaction solution is 8.1 to 8.5.

Preferably, the pH of the PCR reaction solution is 8.1.

Preferably, the ratio of the volume of the sample nucleic acid solution and the volume of the PCR reaction solution is 1:3 to 1:1.

Preferably, in step (2), the volume of the sample nucleic acid solution for adding is 10µL to 20µL.

Preferably, the sample nucleic acid is from HBV, human B27, HPV 6/11, TB or HCMV.

Preferably, the sample nucleic acid comprises HBV nucleic acid; in the nucleic acid extraction solution, the concentration of alkali metal hydroxide is 25 mM, the concentration of Tris-HCl is 1 mM, and the concentration of Chelex-100 is 2% (w/v), and the concentration of EDTA is 0.1% (w/v), the pH of the nucleic acid extraction solution is 11.5; in the PCR reaction solution, the concentration of Tris-HCl is 10 mM, and the pH of the PCR reaction solution is 8.1.

The present invention also provides a sample nucleic acid detection kit suitable for said method of the invention, comprising a nucleic acid extraction solution and a PCR reaction solution, the nucleic acid extraction solution comprising an alkali metal hydroxide 10 mM to 100 mM, Tris-HCl 1 mM to 25 mM, Chelex-100 2% to 5% (w/v), EDTA 0.1% to 0.4% (w/v), the pH of the nucleic acid extraction solution is 10.5 to 12.5; the PCR reaction solution comprises Tris-HCl 5 mM to 75 mM, the pH of the PCR reaction solution is 8.0 to 9.0.

Preferably, the alkali metal hydroxide is NaOH or KOH.

Preferably, the sample nucleic acid detection kit further comprises a concentration solution.

Preferably, the concentration solution comprises NaCl 2.5% to 4.5% (w/w) and PEG6000 10% to 13% (w/v).

Preferably, the pH of the PCR reaction solution is 8.1 to 8.7.

Preferably, the pH of the PCR reaction solution is 8.1 to 8.5.

Preferably, the pH of the PCR reaction solution is 8.1.

Preferably, the sample nucleic acid is from HBV, human B27, HPV 6/11, TB or HCMV.

Preferably, the sample nucleic acid comprises HBV nucleic acid; in the nucleic acid extraction solution, the concentration of alkali metal hydroxide is 25 mM, the concentration of Tris-HCl is 1 mM, and the concentration of Chelex-100 is 2% (w/v) and the concentration of EDTA is 0.1% (w/v), the pH of the nucleic acid extraction solution is 11.5; in the PCR reaction solution, the concentration of Tris-HCl is 10 mM, and the pH of the PCR reaction solution is 8.1.

The present invention also provides the sample nucleic acid detection kit for detecting HBV, human B27 gene, HPV 6/11 type, mycobacterium tuberculosis or human cytomegalovirus in a sample.

The beneficial effect: first adding a certain amount of sample to the strong alkaline nucleic acid extraction solution, extracting the nucleic acid in the sample, and then adding the extracted sample nucleic acid solution to the PCR reaction solution (the pH value is lower than that of the nucleic acid extraction solution) for neutralization, and performing sample nucleic acid amplification and detection, thus eliminating the need to neutralize the extracted sample nucleic acid solution during the sample nucleic acid extraction, thereby effectively saving detection time, reducing the chances of increasing manual errors due to many steps, facilitating semi-automatic or fully-automatic processing, and also increasing the efficiency of PCR amplification of sample nucleic acids and increasing the sensitivity and accuracy of detection of sample nucleic acids. Taking the detection of HBV DNA which may be present in a clinical blood or plasma sample as an example, the present invention improves the nucleic acid extraction solution and the PCR reaction solution in the prior art, and utilizes the nucleic acid extraction solution used in the method of the present invention (pH 10.5 to 12.5) to extract the nucleic acid in the clinical blood or plasma sample to obtain the extracted sample nucleic acid solution, and the extracted sample nucleic acid solution is added to the PCR reaction solution (pH 8.0 to 9.0) used in the method of the present invention for mixing. Regardless of the added extracted sample nucleic acid solution is 2-5µl or 20µl, the pH of the mixed PCR reaction solution will not be affected, that is, will remain in the proper pH range, so as not to affect the subsequent PCR amplification reaction efficiency, and also increase the sensitivity and accuracy of the detection of the sample nucleic acid. In addition, compared to the extraction of the nucleic acid in the sample by using the conventional weak basic nucleic acid lysis solution, using the nucleic acid extraction solution used in the method of the present invention can improve the extraction efficiency of the sample nucleic acid and reduce the impurities and PCR reaction inhibitors which may be present in the extracted sample nucleic acid solution. Further, when the nucleic acid extraction solution used in the method of the present invention is used in combination with the PCR reaction solution used in the method of the present invention, when the volume of the extracted sample nucleic acid solution added to the PCR reaction solution is small (usually 2 to 5 µl), the nucleic acid in the sample can be detected and has a high detection sensitivity; when the volume of the added extracted sample nucleic acid solution is large, if 20 µl of the extracted sample nucleic acid solution is added to 20 µl of the PCR reaction solution for mixing, the sample nucleic acid is detected with highly sensitivity and high accuracy.

### Brief Description of the Drawings

Fig. 1 shows the results of fluorescent PCR detection of different concentrations of HBV positive samples when the pH of the PCR reaction solution is 8.1.
Fig. 2 shows the results of fluorescent PCR detection of different concentrations of HBV positive samples when the pH of the PCR reaction solution is 8.9.
Fig. 3 shows the results of fluorescent PCR detection when the concentration of HBV positive samples is 20 IU/ml, and the pH of the PCR reaction solution is changed, when the pH are 8.1, 8.3, 8.5, 8.7 and 8.9, respectively.

### Detailed description of the Examples

In order to extract the nucleic acid in the sample, in the method of the present invention, a certain amount of the sample is first added to the strong alkaline nucleic acid extraction solution, the cells or viruses in the sample are lysed, and the nucleic acid carried by the cells or viruses is released, thereby being extracted, wherein the pH of the extracted sample nucleic acid solution is relatively high, and will affect the subsequent PCR amplification reaction of the nucleic acid, and even cause failure of the nucleic acid amplification reaction to start. For this purpose, in order to reduce this effect, the extracted sample nucleic acid solution is added to the PCR reaction solution with lower pH for neutralization by the inventors, and the sample nucleic acid amplification and detection are also performed, thereby eliminating the step of neutralizing the extracted sample nucleic acid during sample nucleic acid extraction, effectively saving the detection time, reducing the chance of increasing manual errors due to the many steps, facilitating semi-automatic or fully automated processing, increasing PCR amplification reaction efficiency of the sample nucleic acid, and increasing the sensitivity and accuracy of detection of sample nucleic acids.

In the present invention, the nucleic acid extraction solution for extracting the nucleic acid in the sample may be selected from a high concentration of a strong alkaline solution or a strong alkaline solution containing Chelex-100, and the nucleic acid extraction solution has a pH of ≥ 10, preferably 10.5 to 12.5, so the nucleic acid in the sample can be effectively extracted. The high concentration of strong alkaline solution may be selected from 0.05 M to 0.6 M alkali metal hydroxide solution, preferably 0.05 M to 0.6 M NaOH solution or KOH solution; and a strong alkaline solution containing Chelex-100 includes alkali metal hydroxide 10 mM to 100 mM, Tris-HCl 1 mM to 25 mM, Chelex-100 2% to 5%, and EDTA 0.1% to 0.4%, and the alkali metal hydroxide is preferably NaOH or KOH.

In addition, when the concentration of the target nucleic acid molecule to be detected in the sample is relatively low, it is preferable to add the sample into the concentration solution to concentrate before nucleic acid extraction, and then extract the nucleic acid in the concentrated sample using the nucleic acid extraction solution used in the method of the present invention. As a first specific example of the concentration solution, the concentration solution may include 2.5% to 4.5% of NaCl and 10% to 13% of PEG6000; as a second specific example of the concentration solution, the concentration solution may include 20% of PEG8000, and 13.5% of NaCl.

After extracting the nucleic acid in the sample by using the nucleic acid extraction solution used in the method of the present invention, the extracted sample nucleic acid solution is added to a PCR reaction solution with lower pH in a suitable volume ratio, and the pH of the extracted sample nucleic acid solution is lowered to a level suitable for performing a PCR amplification reaction. In the method of the present invention, the pH of the PCR reaction solution is 8.0 to 9.0, and if the value is too high or too low, it will affect the efficiency of the PCR amplification reaction and reduce the detection sensitivity and accuracy. The PCR reaction solution includes Tris-HCl, KCl, dNTPs and Mg²⁺, etc., wherein Tris-HCl can be replaced by a buffer system such as Tris-Acetate or Tris-Borate, and when the PCR reaction solution is mixed with the extracted sample nucleic acid solution, the concentration of Tris-HCl is ensured to be 5 mM to 75 mM. In addition, the PCR reaction solution may further include a DNA polymerase for performing a nucleic acid amplification reaction such as Taq enzyme, primers and a fluorescent dye such as SYBR Green, or a fluorescent probe such as a TaqMan probe, a two-hybrid probe, a molecular beacon probe, or an MGB probe. Of course, a DNA polymerase, a fluorescent dye or a fluorescent probe can be added to the PCR reaction solution only when a nucleic acid amplification reaction is required.

In the present invention, in order to further reduce non-specific amplification which may occur during PCR amplification, an anti-Taq enzyme antibody may be added to the PCR reaction solution, and the antibody can bind to the active site of the Taq enzyme, thereby making the Taq enzyme unable to function, and only in the denaturing phase, the anti-Taq enzyme antibody is inactivated at high temperature and cannot bind to the active site of the Taq enzyme, so that the Taq enzyme can play a catalytic role to synthesize a new DNA strand.

When the nucleic acid in the sample is extracted by the nucleic acid extraction solution used in the method of the present invention and the nucleic acid in the sample is detected by the PCR amplification reaction, a specific DNA or RNA fragment in the sample nucleic acid is selected in advance, and then at least one pair of specific primers are designed according to such DNA or RNA fragment. If fluorescent PCR amplification is performed, a fluorescent dye such as SYBR Green can be added, or at least one fluorescent probe is designed according to the selected specific DNA or RNA fragment, so that the specific DNA or RNA fragment in the sample is detected according to the fluorescence intensity produced during amplification.

The following examples further illustrate the present invention.

### Example 1: Nucleic acid detection reagent

The sample nucleic acid detection reagent in this example includes a nucleic acid extraction solution and a PCR reaction solution, and the compositions thereof are as follows:
The nucleic acid extraction solution: NaOH 10 mM to 100 mM, Tris-HCl 1 mM to 25 mM, Chelex-100 2% to 5% (w/v), EDTA 0.1% to 0.4% (w/v), pH = 10.5 to 12.5.

The PCR reaction solution (pH = 8.0 to 9.0) includes Tris-HCl 5 mM to 75 mM, and further includes dNTPs, KCl, Mg²⁺ and a pair of primers. When fluorescent PCR detection is used, the PCR reaction solution further includes a fluorescent dye such as SYBR Green, or a fluorescent probe such as TaqMan, preferably a TaqMan fluorescent probe. The PCR reaction solution may further include an enzyme mixture. Of course, the enzyme mixture and the PCR reaction solution may be stored separately, and the enzyme mixture is added to the PCR reaction solution when a nucleic acid amplification reaction is required. When the nucleic acid in the test sample is DNA, the enzyme mixture mainly comprises a DNA polymerase, and the DNA polymerase may be selected from Taq DNA polymerase (abbreviated as Taq enzyme), Vent DNA polymerase, Deep DNA polymerase, Pfu DNA polymerase, Tgo DNA polymerase, and KOD1 DNA polymerase, etc. Taq enzyme is preferable. When the nucleic acid in the test sample is RNA, the enzyme mixture mainly includes MMLV reverse transcriptase, Taq enzyme and RNase inhibitor. When the selected DNA polymerase is Taq enzyme, the enzyme mixture may further include an anti-Taq enzyme antibody, no matter the nucleic acid in the test sample is DNA or RNA.

Preferably, the sample nucleic acid detection reagent further comprises a concentration solution: 2.5% to 4.5% (w/v) of NaCl, and 10% to 13% (w/v) of PEG6000.

When detecting hepatitis B virus (HBV) in serum or plasma samples, concentration solution: NaCl 2.5% (w/v), PEG6000 10% (w/v); nucleic acid extraction solution: NaOH 25 mM, Tris-HCl 1 mM, Chelex-100 2% (w/v), EDTA 0.1% (w/v), pH = 11.5; PCR reaction solution (pH 8.1), wherein the PCR reaction solution includes Tris-HCl 10 mM, and also includes KCl, dNTPs, Mg²⁺, BSA, primers and a TaqMan probe, preferably also includes Taq enzyme.

When detecting the human B27 gene in a whole blood sample, the concentration solution: NaCl 2.5% (w/v), PEG6000 13% (w/v); nucleic acid extraction solution: NaOH 25 mM, Tris-HCl 10 mM, Chelex-100 3% (w/v), EDTA 0.1% (w/v), pH = 12.0; PCR reaction solution, pH 8.8, wherein the PCR reaction solution includes Tris-HCl 5 mM, and also includes KCl, dNTPs, Mg²⁺, BSA, primers and a TaqMan probe, preferably also includes Taq enzyme.

When detecting human papillomavirus type 6/11 (HPV 6/11) in genital tract secretion samples, nucleic acid extraction solution: NaOH 10 mM, Tris-HCl 10 mM, Chelex-100 2% (w/v), EDTA 0.1% (w/v), pH = 10.5; PCR reaction solution, pH 9.0, wherein the PCR reaction solution includes Tris-HCl 75 mM, and also includes KCl, dNTPs, Mg²⁺, BSA, primers and a TaqMan probe, preferably also includes Taq enzyme solution.

When detecting Mycobacterium tuberculosis (TB) in the sputum sample, the concentration solution: NaCl 2.5% (w/v), PEG6000 10% (w/v); nucleic acid extraction solution: NaOH 25 mM, Tris-HCl 25 mM; Chelex-100 2% (w/v); EDTA 0.2% (w/v), pH = 11.5; PCR reaction solution, pH 8.0, wherein the PCR reaction solution includes Tris-HCl 50 mM, and also includes KCl, dNTPs, Mg²⁺, BSA, primers and a TaqMan probe, preferably also includes Taq enzyme.

When detecting human cytomegalovirus (HCMV) in urine samples, concentration solution: NaCl 4.5% (w/v), PEG6000 13% (w/v); nucleic acid extraction solution: NaOH 100 mM, Tris-HCl 10 mM; Chelex-100 5% (w/v), EDTA 0.4% (w/v), pH = 12.5; PCR reaction solution, pH 8.9, wherein the PCR reaction solution includes Tris-HCl 10 mM, and also includes KCl, dNTPs, Mg²⁺, BSA, primers and a TaqMan probe, preferably also includes Taq enzyme.

The NaOH in the above nucleic acid extraction solution may be replaced with other alkali metal hydroxide such as KOH.

### Example 2: Sample nucleic acid extraction step

The following sample nucleic acid extraction is carried out by using the nucleic acid detection reagent in Example 1, and can be classified into the following two sample nucleic acid extraction methods according to the sample:
Scheme 1:
   1. Taking 200 µl of the sample to be tested, adding 200 µl of the concentration solution, and after shaking and mixing, centrifuging at 12,000 rpm for 10 min;
   2. Discarding the supernatant and adding 50 µl of nucleic acid extraction solution to the precipitate;
   3. After shaking and mixing, carrying out 100°C water bath or dry bath for 10 min, then centrifuging at 12,000 rpm for 10 min, taking the supernatant, which is the extracted sample nucleic acid solution for PCR amplification, or storing at -20 °C ± 5 °C for future use.
Scheme 2:
   1. Taking 100 µl of the sample to be tested, and after shaking and mixing, centrifuging at 12,000 rpm for 10 min, discarding the supernatant, and adding 50 µl of the nucleic acid extraction solution to the precipitate;
   2. After shaking and mixing, carrying out 100 °C water bath or dry bath for 10 min, then centrifuging at 12,000 rpm for 10 min, taking the supernatant, which is the extracted sample nucleic acid solution for PCR amplification, or storing at -20 °C ± 5 °C for future use.

In the practical situation, the specific nucleic acid extraction steps are slightly different depending on the sample used for the detection and the test subject to be detected. For example, when detecting hepatitis B virus (HBV) in serum or plasma samples, its nucleic acid extraction step is the scheme 1 of the present example; when detecting the human B27 gene in the whole blood sample, its nucleic acid extraction step is the scheme 1 of the present example; when detecting human papillomavirus type 6/11 (HPV 6/11) in a genital tract secretion sample, its nucleic acid extraction step is the scheme 2 of the present example; when detecting the Mycobacterium tuberculosis (TB) in the sputum sample, its nucleic acid extraction step is the scheme 1 of the present example; when the human cytomegalovirus (HCMV) in the urine sample is detected, its nucleic acid extraction step is the scheme 1 of the present example.

### Example 3: Fluorescent PCR detection

The PCR detection system was 40 µl, the PCR reaction solution was dispensed into PCR reaction tubes in portions per person, and transferred to the sample processing area. 4 to 20 µl of the sample nucleic acid solution extracted in Example 2 was respectively added to the corresponding reaction tubes, and the reaction tubes were tightly capped, and transferred to a detection zone for PCR amplification. Each reaction tube was placed in a PCR machine in a certain order, and PCR detection program and detection fluorescence were set for fluorescence PCR amplification.

When detecting hepatitis B virus (HBV) in serum or plasma samples, 40 µl of a PCR detection system was prepared, in which the extracted sample nucleic acid solution was 20 µl and the PCR reaction solution was 20 µl.

When detecting the human B27 gene in the whole blood sample, the PCR detection system was 40 µl, in which the extracted sample nucleic acid solution was 4 µl and the PCR reaction solution was 36 µl.

When detecting human papillomavirus type 6/11 (HPV 6/11) in the genital tract secretion sample, the PCR detection system was 40 µl, wherein the extracted sample nucleic acid solution was 4 µl and the PCR reaction solution was 36 µl.

When detecting mycobacterium tuberculosis (TB) in the sputum sample, the PCR detection system was 40 µl, wherein the extracted sample nucleic acid solution was 10 µl and the PCR reaction solution was 30 µl.

When detecting human cytomegalovirus (HCMV) in a urine sample, the PCR detection system was 40 µl, wherein the extracted sample nucleic acid solution was 4 µl and the PCR reaction solution was 36 µl.

### Example 4: Clinical sample HBV detection

The type of sample is serum or plasma, and the detection of hepatitis B virus (HBV) is taken as an example. According to the nucleic acid detection reagent, the nucleic acid extraction method and the fluorescent PCR detection of Example 1, Example 2 and Example 3, 20 clinical samples clinically confirmed to be positive or negative were detected.

Control method: adding 50 µl of the sample to be tested to 50 µl of nucleic acid extraction solution (NaCl 2 M, Tris-HCl (pH 8.1) 50 mM, mercaptoethanol 1% (v/v), Chelex-100 5% (w/v), pH 8.59), shaking and mixing for 15 sec, carrying out 100 °C water bath or dry bath for 10 min, and then centrifuging at 12,000 rpm for 10 min, and respectively taking 4 µl and 20 µl of the supernatant for PCR amplification. The PCR reaction system includes a PCR reaction solution (containing TAKARA PCR buffer, dNTPs, Mg²⁺, primers and a TaqMan probe, pH 9.05) and Taq enzyme. The detection sensitivity of this control method was 500 IU/ml.

Table 1 shows the detection results of HBV in serum or plasma samples, where "+" indicates a positive result and "-" indicates a negative result.

**Table 1 Detection results of HBV in serum or plasma samples**

| Sample No. | The detection result of the present invention | The detection result of the control method | | Clinical sample information |
|---|---|---|---|---|
| | | 4 µl | 20 µl | |
| 1 | + | + | - | + |
| 2 | + | + | - | + |
| 3 | + | + | - | + |
| 4 | + | - | - | + |
| 5 | + | + | - | + |
| 6 | + | + | - | + |
| 7 | + | + | - | + |
| 8 | + | + | - | + |
| 9 | + | + | - | + |
| 10 | + | + | - | + |
| 11 | + | + | - | + |
| 12 | - | - | - | - |
| 13 | - | - | - | - |
| 14 | - | - | - | - |
| 15 | - | - | - | - |
| 16 | - | - | - | - |
| 17 | - | - | - | - |
| 18 | - | - | - | - |
| 19 | - | - | - | - |
| 20 | - | - | - | - |

The experimental results show that in the 20 detection samples, 11 HBV positive cases and 9 HBV negative cases were detected by the nucleic acid detection reagent, the nucleic acid extraction method and the fluorescent PCR detection used in the present invention. For the control method, when 4 µl of the supernatant was used for the detection, 10 HBV positive cases and 10 HBV negative cases were detected, and one of the control detections was inconsistent with the clinical sample information; when 20 µl of the supernatant was used for detection, the detection results of the 20 test samples were all negative, because the pH of the nucleic acid extraction reagent in the control method was not highly alkaline relative to the method of the present invention, and the nucleic acid extraction efficiency was not high. In addition, when the sample amount was increased to 20 µl, the substance inhibiting PCR amplification was also possibly increased accordingly.

### Example 5: Effect of pH of PCR reaction solution on sensitivity of HBV detection

According to the nucleic acid detection reagent, the nucleic acid extraction method and the fluorescent PCR detection of Example 1, Example 2 and Example 3, the HBV positive samples were detected: high-concentration HBV positive samples were serially diluted respectively to obtain a series of low-concentration HBV positive samples, the concentrations ranging from 1000 IU/ml, 500 IU/ml, 250 IU/ml, 100 IU/ml, 50 IU/ml and 20 IU/ml, respectively.

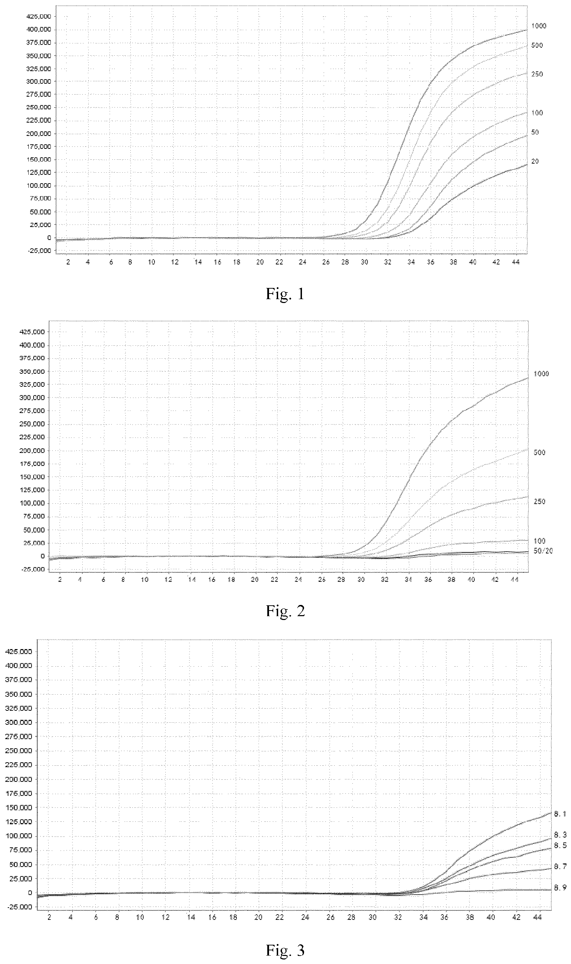

The pH of the PCR reaction solution was adjusted to 8.1, and the results of fluorescent PCR detection for these HBV positive samples were shown in Fig. 1. The results showed that the six low-concentration HBV positive samples all had obvious S-type amplification curves and were all stably detected.

When the pH of the PCR reaction solution was adjusted to 8.9, the results of fluorescent PCR detection for these HBV positive samples were shown in Fig. 2. The results showed that in the six low-concentration HBV positive samples, positive samples at concentrations of 1000 IU/ml, 500 IU/ml and 250 IU/ml were stably detected, while positive samples at concentrations of 100 IU/ml, 50 IU/ml and 20 IU/ml could not be detected stably. It can be seen that when the pH of the PCR reaction solution used in the method of the present invention was 8.9, its detection sensitivity was 250 IU/ml, which was also superior to 500 IU/ml in the control method.

In addition, for the HBV positive samples with a concentration of 20 IU/ml, the pH of the PCR reaction solution was adjusted to 8.1, 8.3, 8.5, 8.7 and 8.9, respectively, and the results of the fluorescent PCR detection were shown in Fig. 3. The results showed that the amplification efficiency of the HBV positive samples with a concentration of 20 IU/ml increased significantly with the decrease of the pH of the PCR reaction solution. When the pH was 8.1, the amplification efficiency was the best. The results also showed that when the pH values of the PCR reaction solutions were 8.1, 8.3, 8.5 and 8.7 respectively, positive samples of 20 IU/ml could be detected, but the amplification efficiency, the linearity and fluorescence values of S-type amplification curve at pH of 8.1, 8.3 and 8.5 respectively were significantly better than those at the pH 8.7; the amplification efficiency, the linearity and fluorescence values of S-type amplification curve at pH 8.1 were significantly better than those at the pH values of 8.3 and 8.5. It can be seen that in the case that other detection conditions were the same, change of the pH values can have a very large influence on the amplification efficiency, the linearity and fluorescence values of an S amplification curve, while the increase of the amplification efficiency and the fluorescence values of the S-type amplification curve can increase the sensitivity of the detection, so the detection sensitivity at pH values of 8.1, 8.3, and 8.5 is significantly better than that at pH 8.7; the detection sensitivity at pH 8.1 is significantly better than that at the pH values of 8.3 and 8.5. This means that while the pH value gradually decreases, the detection sensitivity gradually increases.

For the detection of HBV DNA in serum or plasma, the present inventors unexpectedly found that when the pH of the PCR reaction solution adjusted from 8.9 to 8.7, from 8.7 to 8.3-8.5, and from 8.3 to 8.1, an unexpected amplification effect was obtained, thus significantly improving the detection accuracy.

### Example 6: Clinical sample human B27 gene detection

The clinical detection with whole blood as the sample type takes the human B27 gene detection as an example. According to the nucleic acid detection reagent, the nucleic acid extraction method and the fluorescent PCR detection of Example 1, Example 2 and Example 3, 13 clinical samples clinically confirmed to be positive or negative were detected.

Control method: 500 µl whole blood sample was pretreated with red blood cell lysate (NH₄Cl 15 mM, NaHCO₃ 1 mM, EDTA-2Na 0.1 mM), and after centrifugation, the supernatant was removed, and the precipitate was retained; 100 µl of nucleic acid extraction solution (Tris-HCl (pH 8.0) 100 mM, Chelex-100 5%, EDTA (pH 8.0) 10 mM, NaCl 50 mM, SDS 2% (w/v), pH 8.2) was added to the precipitate, shaken and mixed for 15 sec, subjected to 100 °C water bath or dry bath for 10 min, then centrifuged at 12,000 rpm for 10 min, and 4 µl of the supernatant was taken for PCR amplification. The PCR reaction system includes a PCR reaction solution (containing TAKARA PCR buffer, dNTPs, Mg²⁺, primers and a TaqMan probe, pH 9.05) and Taq enzyme.

Table 2 shows the detection results of human B27 gene in whole blood samples, where "+" indicates a positive result and "-" indicates a negative result.

**Table 2 Detection results of human B27 gene in whole blood samples**

| Sample No. | The detection result of the present invention | The detection result of the control method | Clinical sample information |
|---|---|---|---|
| 1 | + | + | + |
| 2 | + | + | + |
| 3 | + | + | + |
| 4 | + | + | + |
| 5 | + | - | + |
| 6 | + | + | + |
| 7 | - | - | - |
| 8 | - | - | - |
| 9 | - | - | - |
| 10 | - | - | - |
| 11 | - | - | - |
| 12 | - | - | - |
| 13 | - | - | - |

The experimental results show that in the 13 test samples, 6 positive human B27 genes and 7 negative human B27 genes were detected by using the nucleic acid detection reagent, nucleic acid extraction method and fluorescent PCR detection used in the present invention; five positive human B27 genes, 8 negative human B27 genes were detected by control method, and one of the control detections was inconsistent with the clinical sample information.

### Example 7: Clinical sample HPV6/11 detection

The clinical detection with genital tract secretions as the sample type takes human papillomavirus type 6/11 (HPV 6/11) detection as an example. According to the nucleic acid detection reagent, the nucleic acid extraction method and the fluorescent PCR detection of Example 1, Example 2 and Example 3, 15 clinical samples clinically confirmed to be positive or negative were detected.

Control method: 50 µl of the sample to be tested was added to 50 µl of nucleic acid extraction solution (NaCl 2 M, Tris-HCl (pH 8.0) 50 mM, mercaptoethanol 1% (v/v), Chelex-100 5% (w/v), pH 8.59), shaken and mixed for 15 sec, subjected to 100 °C water bath or dry bath for 10 min, then centrifuged at 10,000 rpm for 5 min, and 4 µl of the supernatant was taken for PCR amplification. The PCR reaction system includes a PCR reaction solution (containing TAKARA PCR buffer, dNTPs, Mg²⁺, primers and a TaqMan probe, pH 9.05) and a Taq enzyme.

Table 3 shows the detection results of HPV in samples of genital tract secretions, where "+" indicates a positive result and "-" indicates a negative result.

**Table 3 Detection results of HPV gene in genital tract secretion samples**

| Sample No. | The detection result of the present invention | The detection result of the control method | Clinical sample information |
|---|---|---|---|
| 1 | + | + | + |
| 2 | + | - | + |
| 3 | + | + | + |
| 4 | + | - | + |
| 5 | + | + | + |
| 6 | + | + | + |
| 7 | + | + | + |
| 8 | + | + | + |
| 9 | + | + | + |
| 10 | + | + | + |
| 11 | - | - | - |
| 12 | - | - | - |
| 13 | - | - | - |
| 14 | - | - | - |
| 15 | - | - | - |

The experimental results show that in the 15 test samples, 10 positive nucleic acid samples and 5 HPV negative samples were detected by the nucleic acid detection reagent, nucleic acid extraction method and fluorescent PCR detection used in the present invention; 8 positive samples and 7 negative samples were detected by the control method, and 2 of the control group were inconsistent with the clinical sample information.

### Example 8: Clinical sample TB detection

The clinical detection with sputum as the sample type takes the detection of mycobacterium tuberculosis (TB) detection as an example. According to the nucleic acid detection reagent, nucleic acid extraction methods and fluorescent PCR detection of Example 1, Example 2 and Example 3, 10 samples clinically confirmed to be positive or negative were detected.

Control method: 500 µl sputum sample was added to 5 ml of 1 M NaOH solution, and after liquefied and shaken for 30 min, centrifuged at 14,000 for 5 min, and the supernatant was removed, and the precipitate was retained; 50 µl of nucleic acid extraction solution (SDS 0.1% (w/v), NP40 1% (v/v), Tween-20 1% (v/v), Chelex-100 15% (w/v), pH 5.02) was added to the precipitate, shaken and mixed for 15 sec, subjected to 100 °C water bath or dry bath for 30 min, then centrifuged at 12,000 rpm for 10 min, and 10 µl of the supernatant was taken for PCR amplification. The PCR reaction system includes a PCR reaction solution (containing TAKARA PCR buffer, dNTPs, Mg²⁺, primers and a TaqMan probe, pH 9.05) and a Taq enzyme.

Table 4 shows the detection results of TB in sputum samples, where "+" indicates a positive result and "-" indicates a negative result.

**Table 4 Detection results of TB in sputum samples**

| Sample No. | The detection result of the present invention | The detection result of the control method | Clinical sample information |
|---|---|---|---|
| 1 | + | + | + |
| 2 | + | - | + |
| 3 | + | + | + |
| 4 | + | + | + |
| 5 | + | + | + |
| 6 | - | - | - |
| 7 | - | - | - |
| 8 | - | - | - |
| 9 | - | - | - |
| 10 | - | - | - |

The experimental results show that in the 10 test samples, 5 TB positive and 5 TB negative samples were detected by the nucleic acid detection reagent, nucleic acid extraction method and fluorescent PCR detection used in the present invention; 4 positive, 6 negative samples were detected by the control method, and one sample result of the control was inconsistent with the clinical sample information.

### Example 9: Clinical sample HCMV detection

The clinical detection with urine as the sample type takes the human cytomegalovirus (HCMV) detection as an example. According to the nucleic acid detection reagent, the nucleic acid extraction method and the fluorescent PCR detection of Example 1, Example 2 and Example 3, 10 clinical samples clinically confirmed to be positive or negative were detected.

Control method: 250 µl of the urine sample to be tested was collected, centrifuged at 14,000 for 5 min, the supernatant was removed, and the precipitate was retained; 250 µl of nucleic acid extraction solution (Tris-HCl (pH 8.0) 100 mM, Chelex-100 5%, EDTA (pH 8.0) 10 mM, NaCl 50 mM, SDS 2% (w/v), pH 8.2) was added to the precipitate, shaken and mixed for 15 sec, subjected to 100 °C water bath or dry bath for 10 min and then centrifuged at 13,000 rpm for 10 min, and 4 µl of the supernatant was taken for PCR amplification. The PCR reaction system includes a PCR reaction solution (containing TAKARA PCR buffer, dNTPs, Mg²⁺, primers and a TaqMan probe, pH 9.05) and a Taq enzyme.

Table 5 shows the results of HCMV detection in urine samples, where "+" indicates a positive result and "-" indicates a negative result.

**Table 5 Detection results of HCMV in urine samples**

| Sample No. | The detection result of the present invention | The detection result of the control method | Clinical sample information |
|---|---|---|---|
| 1 | + | + | + |
| 2 | + | + | + |
| 3 | + | + | + |
| 4 | + | + | + |
| 5 | + | + | + |
| 6 | - | - | - |
| 7 | - | - | - |
| 8 | - | - | - |
| 9 | - | - | - |
| 10 | - | - | - |

The experimental results show that in the 10 test samples, 5 nucleic acid positive samples and 5 HCMV negative samples were detected by the nucleic acid detection reagent, nucleic acid extraction method and fluorescent PCR detection used in the present invention; 5 positive samples and 5 negative samples were detected by the control method; the two detection results were consistent.

### Example 10: Sensitivity detection result

According to the nucleic acid detection reagent, nucleic acid extraction method and fluorescent PCR detection of Example 1, Example 2 and Example 3, HBV, human B27 gene, HPV, TB, HCMV positive standard samples were detected: the positive standard samples were diluted respectively, diluted according to Table 6 respectively, and each concentration is repeated 8 times. The detection results are shown in Table 6, in which "+" indicates a positive result and "-" indicates lower than a lower limit of the reagent detection.

**Table 6 Sensitivity detection results**

| Sam ple | Sensitivity detection | | | | | | | | | | | | | | |
|---|---|---|---|---|---|---|---|---|---|---|---|---|---|---|---|
| | HBV (IU/ml) | | | B27 gene (ng/reaction) | | | HPV 6/11 (copies/ml) | | | TB (bacterium /ml) | | | HCMV (copies/ml) | | |
| Conc entra tion | 50 | 20 | 10 | 10 | 5 | 2. 5 | 500 | 400 | 250 | 10 | 5 | 1 | 1000 | 500 | 250 |
| 1 | + | + | + | + | + | + | + | + | + | + | + | + | + | + | + |
| 2 | + | + | + | + | + | + | + | + | + | + | + | - | + | + | + |
| 3 | + | + | + | + | + | + | + | + | + | + | + | + | + | + | + |
| 4 | + | + | - | + | + | + | + | + | + | + | + | + | + | + | + |
| 5 | + | + | + | + | + | + | + | + | + | + | + | - | + | + | + |
| 6 | + | + | + | + | + | - | + | + | + | + | + | + | + | + | + |
| 7 | + | + | - | + | + | + | + | + | + | + | + | - | + | + | + |
| 8 | + | + | + | + | + | + | + | + | + | + | + | + | + | + | - |

The above results show that the HBV detection sensitivity of the present invention is 20 IU/ml, the detection sensitivity of human B27 gene is 5 ng/reaction, the HPV6/11 detection sensitivity is 250 copies/ml, the TB detection sensitivity is 5 bacteria/ml, and the HCMV detection sensitivity is 500 copies/ml.

## Claims

1. A method for detecting nucleic acid in a sample, which method comprises:
(1) extracting nucleic acid in a sample by using a nucleic acid extraction solution to obtain a sample nucleic acid solution;
(2) adding the sample nucleic acid solution to a PCR reaction solution for neutralization to obtain a mixed solution, using the mixed solution to perform amplification reaction and to detect nucleic acid in the sample, wherein the nucleic acid extraction solution comprises an alkali metal hydroxide 10 mM to 100 mM, Tris-HCl 1 mM to 25 mM, Chelex-100 2% to 5% (w/v), EDTA 0.1% to 0.4% (w/v), the pH of the nucleic acid extraction solution is 10.5 to 12.5; the PCR reaction solution comprises Tris-HCl 5 mM to 75 mM, the pH of the PCR reaction solution is 8.0 to 9.0; and the pH of the mixed solution is 8.0 to 9.0.

2. The method according to claim 1, wherein in step (2), the ratio of the volume of the sample nucleic acid solution and the volume of the PCR reaction solution is 1:3 to 1:1.

3. The method according to claim 1, wherein in step (2), the volume of the sample nucleic acid solution for adding is 10 µL to 20 µL.

4. The method according to claim 1, wherein there is no extra neutralization step for neutralizing the sample nucleic acid solution between step (1) and step (2).

5. The method according to claim 1, wherein the PCR reaction solution comprises Tris-HCl 50 mM to 75 mM.

6. The method according to claim 1, wherein the sample nucleic acid is from HBV, human B27, HPV 6/11, TB or HCMV.

7. A sample nucleic acid detection kit suitable for the method of claim 1 comprising a nucleic acid extraction solution and a PCR reaction solution, wherein the nucleic acid extraction solution comprises an alkali metal hydroxide 10 mM to 100 mM, Tris-HCl 1 mM to 25 mM, Chelex-100 2% to 5% (w/v), EDTA 0.1% to 0.4% (w/v), the pH of the nucleic acid extraction solution is 10.5 to 12.5; the PCR reaction solution includes Tris-HCl 5 mM to 75 mM, the pH of the PCR reaction solution is 8.0 to 9.0, preferably wherein the pH of the PCR reaction solution is 8.1 to 8.7, more preferably wherein the pH of the PCR reaction solution is 8.1 to 8.5.

8. The sample nucleic acid detection kit according to claim 7, wherein the alkali metal hydroxide is NaOH or KOH.

9. The sample nucleic acid detection kit according to claim 7, wherein the PCR reaction solution comprises Tris-HCl 50 mM to 75 mM.

10. The sample nucleic acid detection kit according to claim 7, wherein the sample nucleic acid detection kit further comprises a concentration solution comprising 2.5% to 4.5% (w/w) of NaCl and 10% to 13% (w/v) of PEG6000.

11. The sample nucleic acid detection kit according to claim 7, wherein the sample nucleic acid is from HBV, human B27, HPV 6/11, TB or HCMV.

## Patentansprüche

1. Verfahren zum Nachweisen von Nukleinsäure in einer Probe, wobei das Verfahren umfasst:
(1) Extrahieren von Nukleinsäure in einer Probe unter Verwendung einer Nukleinsäureextraktionslösung, um eine Probennukleinsäurelösung zu erhalten;
(2) Zugeben der Probennukleinsäurelösung zu einer PCR-Reaktionslösung zur Neutralisierung, um eine gemischte Lösung zu erhalten, Verwenden der gemischten Lösung zum Durchführen einer Amplifikationsreaktion und zum Nachweisen von Nukleinsäure in der Probe, wobei die Nukleinsäureextraktionslösung 10 mM bis 100 mM eines Alkalimetallhydroxids, 1 mM bis 25 mM Tris-HCl, 2 Gew.-% bis 5 Gew.-% Chelex-100, 0,1 Gew.-% bis 0,4 Gew.-% EDTA umfasst, wobei der pH-Wert der Nukleinsäureextraktionslösung 10,5 bis 12,5 beträgt; wobei die PCR-Reaktionslösung 5 mM bis 75 mM Tris-HCl umfasst, wobei der pH-Wert der PCR-Reaktionslösung 8,0 bis 9,0 beträgt; und der pH-Wert der gemischten Lösung 8,0 bis 9,0 beträgt.

2. Verfahren nach Anspruch 1, wobei das Verhältnis des Volumens der Probennukleinsäurelösung und des Volumens der PCR-Reaktionslösung in Schritt (2) 1:3 bis 1:1 beträgt.

3. Verfahren nach Anspruch 1, wobei das Volumen der Probennukleinsäurelösung für das Zugeben in Schritt (2) 10 µl bis 20 µl beträgt.

4. Verfahren nach Anspruch 1, wobei zwischen Schritt (1) und Schritt (2) kein zusätzlicher Neutralisierungsschritt zum Neutralisieren der Probennukleinsäurelösung erfolgt.

5. Verfahren nach Anspruch 1, wobei die PCR-Reaktionslösung 50 mM bis 75 mM Tris-HCl umfasst.

6. Verfahren nach Anspruch 1, wobei die Probennukleinsäure aus HBV, humanem B27, HPV 6/11, TB oder HCMV stammt.

7. Nachweiskit für Probennukleinsäure, geeignet für das Verfahren nach Anspruch 1, umfassend eine Nukleinsäureextraktionslösung und eine PCR-Reaktionslösung, wobei die Nukleinsäureextraktionslösung 10 mM bis 100 mM eines Alkalimetallhydroxids, 1 mM bis 25 mM Tris-HCl, 2 Gew.-% bis 5 Gew.-% Chelex-100, 0,1 Gew.-% bis 0,4 Gew.-% EDTA umfasst, wobei der pH-Wert der Nukleinsäureextraktionslösung 10,5 bis 12,5 beträgt; wobei die PCR-Reaktionslösung 5 mM bis 75 mM Tris-HCl umfasst, wobei der pH-Wert der PCR-Reaktionslösung 8,0 bis 9,0 beträgt; wobei der pH-Wert der PCR-Reaktionslösung vorzugsweise 8,1 bis 8,7 beträgt, wobei der pH-Wert der PCR-Reaktionslösung bevorzugter 8,1 bis 8,5 beträgt.

8. Nachweiskit für Probennukleinsäure nach Anspruch 7, wobei das Alkalimetallhydroxid NaOH oder KOH ist.

9. Nachweiskit für Probenukleinsäure nach Anspruch 7, wobei die PCR-Reaktionslösung 50 mM bis 75 mM Tris-HCl umfasst.

10. Nachweiskit für Probennukleinsäure nach Anspruch 7, wobei das Nachweiskit für Probennukleinsäure ferner eine Konzentrationslösung umfasst, die 2,5 Gew.-% bis 4,5 Gew.-% NaCl und 10 Gew.-% bis 13 Gew.-% PEG6000 umfasst.

11. Nachweiskit für Probennukleinsäure nach Anspruch 7, wobei die Probennukleinsäure aus HBV, humanem B27, HPV 6/11, TB oder HCMV stammt.

## Revendications

1. Procédé pour détecter un acide nucléique dans un échantillon, lequel procédé comprend :
(1) l'extraction d'acide nucléique dans un échantillon en utilisant une solution d'extraction d'acide nucléique pour obtenir une solution d'acide nucléique d'échantillon ;
(2) l'ajout de la solution d'acide nucléique d'échantillon à une solution de réaction de PCR pour neutralisation afin d'obtenir une solution mélangée, l'utilisation de la solution mélangée pour effectuer une réaction d'amplification et pour détecter l'acide nucléique dans l'échantillon, dans lequel la solution d'extraction d'acide nucléique comprend un hydroxyde de métal alcalin 10 mM à 100 mM, du Tris-HCl 1 mM à 25 mM, du Chelex-100 2 % à 5 % (p/v), de l'EDTA 0,1 % à 0,4 % (p/v), le pH de la solution d'extraction d'acide nucléique est de 10,5 à 12,5 ; la solution de réaction de PCR comprend du Tris-HCl 5 mM à 75 mM, le pH de la solution de réaction de PCR est de 8,0 à 9,0 ; et le pH de la solution mélangée est de 8,0 à 9,0.

2. Procédé selon la revendication 1, dans lequel, dans l'étape (2), le rapport du volume de la solution d'acide nucléique d'échantillon et du volume de la solution de réaction de PCR est de 1:3 à 1:1.

3. Procédé selon la revendication 1, dans lequel, dans l'étape (2), le volume de la solution d'acide nucléique d'échantillon à ajouter est de 10 µL à 20 µL.

4. Procédé selon la revendication 1, dans lequel il n'y a pas d'étape de neutralisation supplémentaire pour neutraliser la solution d'acide nucléique d'échantillon entre l'étape (1) et l'étape (2).

5. Procédé selon la revendication 1, dans lequel la solution de réaction de PCR comprend du Tris-HCl 50 mM à 75 mM.

6. Procédé selon la revendication 1, dans lequel l'acide nucléique d'échantillon provient de VHB, B27 humain, HPV 6/11, TB ou CMVH.

7. Kit de détection d'acide nucléique d'échantillon approprié pour le procédé selon la revendication 1, comprenant une solution d'extraction d'acide nucléique et une solution de réaction de PCR, dans lequel la solution d'extraction d'acide nucléique comprend un hydroxyde de métal alcalin 10 mM à 100 mM, du Tris-HCl 1 mM à 25 mM, du Chelex-100 2 % à 5 % (p/v), de l'EDTA 0,1 % à 0,4 % (p/v), le pH de la solution d'extraction d'acide nucléique est de 10,5 à 12,5 ; la solution de réaction de PCR comprend du Tris-HCl 5 mM à 75 mM, le pH de la solution de réaction de PCR est de 8,0 à 9,0, de préférence dans lequel le pH de la solution de réaction de PCR est de 8,1 à 8,7, plus préférablement dans lequel le pH de la solution de réaction de PCR est de 8,1 à 8,5.

8. Kit de détection d'acide nucléique d'échantillon selon la revendication 7, dans lequel l'hydroxyde de métal alcalin est NaOH ou KOH.

9. Kit de détection d'acide nucléique d'échantillon selon la revendication 7, dans lequel la solution de réaction de PCR comprend du Tris-HCl 50 mM à 75 mM.

10. Kit de détection d'acide nucléique d'échantillon selon la revendication 7, dans lequel le kit de détection d'acide nucléique d'échantillon comprend en outre une solution de concentration comprenant 2,5 % à 4,5 % (p/p) de NaCl et 10 % à 13 % (p/v) de PEG6000.

11. Kit de détection d'acide nucléique d'échantillon selon la revendication 7, dans lequel l'acide nucléique d'échantillon provient de HBV, B27 humain, HPV 6/11, TB ou CMVH.
